# EUROPEAN PATENT APPLICATION

(11) **EP 4 403 650 A1**
(43) Date of publication of application: **24.07.2024**
(21) Application number: 23156490.7
(22) Date of filing: 14.02.2023
(51) Int. Cl.: C12Q 1/689, C12Q 1/6844

(54) **NUCLEIC ACID DETECTION REAGENT AND DETECTION METHOD FOR MYCOBACTERIUM TUBERCULOSIS**

(30) Priority: 18.01.2023 US 202318098196
(71) Applicant: Anbio Biotechnology Limited, Road Town VG1110 Tortola (VG); Wang, Jincheng, Xiamen City Fujian (CN)
(72) Inventor: WANG, Daming, Xiamen City (CN); WANG, Jincheng, Xiamen City, Fujian (CN)
(74) Representative: Sach, Greg Robert

(57) **Abstract**

The present application provides a nucleic acid detection reagent and detection method for Mycobacterium tuberculosis, comprising: Tris-HCL, MgCl2, UNG enzyme, Bst DNA polymerase, reverse transcriptase, trehalose, sucrose, mannitol, Tween 20, guanidine hydrochloride and the primers of six kinds of Mycobacterium tuberculosis, the primers of six kinds of Mycobacterium tuberculosis are TB-F3, TB-R3, TB-FIP, TB-BIP, TB-LF and TB-LB, wherein TB is tuberculosis branch bacilli. In the embodiment of the present application, the nucleic acid detection reagent can be used to perform a one-step detection method for the nucleic acid (DNA or RNA) of pathogens present in throat swabs, nasal swabs, saliva, etc., thereby realizing a convenient, efficient and rapid detection, to achieve the sample in, the result out.

## Description

### TECHNICAL FIELD

The present application relates to the field of biotechnology, specifically a nucleic acid detection reagent and detection method for Mycobacterium tuberculosis.

### BACKGROUND

Tuberculosis has been one of the most important public health issues in the world since its discovery, and it seriously endangers human life. According to the World Health Organization (WHO) 2019 Global Tuberculosis Report, there were about 10 million new tuberculosis patients in 2018, and about 1.45 million of them died of ineffective treatment. Among these cases, the proportion of male patients was significantly higher than that of females and children. In order to control the epidemic of tuberculosis, WHO proposed the "End Tuberculosis Strategy" in 2015. The goal of this strategy is to reduce the number of deaths caused by tuberculosis by 95% and the number of cases by 90% in 2035 compared with 2015. However, with the prevalence of AIDS, the difficulty of tuberculosis control is increasing. It is reported that 8.6% of tuberculosis patients are co-infected with HIV. In addition, the emergence of multidrug-resistant and rifampicin-resistant tuberculosis (MDR/RR-TB) is increasing year by year, which brings new challenges to the treatment and control of tuberculosis.

For a long time, the routine laboratory diagnosis of tuberculosis still mainly relies on acid-fast staining, culture, etc. The positive rate detected by these methods is low, the specificity is poor and the time-consuming is long. Not conducive to the rapid diagnosis of tuberculosis. Therefore, in order to effectively control tuberculosis, it is necessary to find new markers and new laboratory diagnostic methods for tuberculosis.

The main challenges in the current laboratory diagnosis of tuberculosis include the difficulty in distinguishing latent infection from active tuberculosis, the difficulty in predicting which patients with latent infection may develop active tuberculosis in the future, and the lack of effective indicators for monitoring tuberculosis treatment.

In view of this, it is an urgent problem to be solved in this technical field to overcome the shortcomings of the products in the prior art.

### SUMMARY OF THE APPLICATION

The technical problem mainly solved by the present application is to provide a nucleic acid detection reagent and detection method for Mycobacterium tuberculosis. The nucleic acid detection reagent can be used to perform a one-step detection method for the nucleic acid (DNA or RNA) of pathogens present in throat swabs, nasal swabs, saliva, etc., thereby realizing a convenient, efficient and rapid Detection, to achieve the sample in, the result out.

In order to solve the above technical problems, a technical solution provided by the application is: A nucleic acid detection reagent for Mycobacterium tuberculosis, comprising: Tris-HCL, MgCl2, UNG enzyme, Bst DNA polymerase, reverse transcriptase, trehalose, sucrose, mannitol, Tween 20, guanidine hydrochloride and the primers of six kinds of Mycobacterium tuberculosis, the primers of six kinds of Mycobacterium tuberculosis are TB-F3, TB-R3, TB-FIP, TB-BIP, TB-LF and TB-LB, wherein TB is tuberculosis branch bacilli.

Further, the sequence information of TB-F3 is ATACGGCCCAGAC; the sequence information of TB-R3 is GTAATTCCGGACAACG; the sequence information of TB-FIP is TACAACCCGAAGGCCGTCATCAGTGGGGAATAT; the sequence information of TB-BIP is TCCGGGTTCTCTCGGATTGAC ACCCTACGTATT; the sequence information of TB-LF is ATCAGGCTTGCGCC, and the sequence information of TB-LB is AACTACGTGCCAGCAG.

Further, the primers are designed according to genome nucleic acid sequence of Mycobacterium tuberculosis, so as to obtain the sequence information of the primers.

Further, a concentration of TB-F3 is 100 µmol/L, a concentration of TB-R3 is 100 µmol/L, and a concentration of TB-FIP is 100 µmol/L, a concentration of TB-BIP is 100 µmol/L, a concentration of TB-LF is 100 µmol/L and a concentration of TB-LB is 100 µmol/L.

Further, a volume of TB-F3 is 0.030 µL-0.050 µL, a volume of TB-R3 is 0.030 µL-0.050 µL, and a volume of TB-FIP is 0.800 µL - 1.300 µL, a volume of TB-BIP is 0.800 µL-1.300 µL, a volume of TB-LF is 0.050 µL-0.080 µL and a volume of TB-LB is 0.050 µL-0.080 µL.

Further, a concentration of Tris-HCL is 200 mmol/L, a concentration of MgCl2 is 25mmol/L, a concentration of UNG enzyme is 1 U/µL, a concentration of Bst DNA polymerase is 8000 U/mL, a concentration of reverse transcriptase is 15000 units/mL, a concentration of trehalose is 3 mmol/L, a concentration of sucrose is 5 mmol/L, a concentration of mannitol is 2 mmol/L, a concentration of guanidine hydrochloride is 3 mmol/L.

Further, a volume of Tris-HCL is 1.5-2 µL, a volume of MgCl2 is 1-1.5 µL, a volume of UNG enzyme is 0.02-0.05 µL, a volume of Bst DNA polymerizes is 0.3-0.5 µL, a volume of reverse transcriptase is 0.3-0.5 µL, a volume of trehalose is 0.1-0.2 µL, a volume of sucrose is 0.05-0.1 µL, a volume of mannitol is 2-6 µL, a volume of guanidine hydrochloride is 0.05-0.08 µL.

Further, the nucleic acid detection reagent is freeze-dried and stored at room temperature.

Further, the raw material of the nucleic acid detection reagent further includes a fluorescent reagent or a fluorescent probe.

In order to solve the above technical problems, a technical solution adopted by the present application is to provide a nucleic acid detection method for Mycobacterium tuberculosis, wherein a detection of Mycobacterium tuberculosis based on the nucleic acid detection reagent according to claim 1 comprising:
put the freeze-dried nucleic acid detection reagent of Mycobacterium tuberculosis into a detection device;
take 25 µL-40 µL of a sample to be tested and add it to the detection device;
turn on a heating switch, raise the temperature to 60°C∼70°C, react at a constant temperature for 20-35 min, and automatically heat up to react, when a preset reaction time is reached or the reaction is over, start a cooling fan to dissipate heat;
observe the situation at bottom of a reaction tube displayed in a reflector through an observation window to draw the detection conclusion.

The beneficial effects of the present application are: the present application provides a nucleic acid detection reagent and detection method for Mycobacterium tuberculosis, comprising: Tris-HCL, MgCl2, UNG enzyme, Bst DNA polymerase, reverse transcriptase, trehalose, sucrose, mannitol, Tween 20, guanidine hydrochloride and the primers of six kinds of Mycobacterium tuberculosis, the primers of six kinds of Mycobacterium tuberculosis are TB-F3, TB-R3, TB-FIP, TB-BIP, TB-LF and TB-LB, wherein TB is tuberculosis branch bacilli.

In the embodiment of the present application, the nucleic acid detection reagent can be used to perform a one-step detection method for the nucleic acid (DNA or RNA) of pathogens present in throat swabs, nasal swabs, saliva, etc., thereby realizing a convenient, efficient and rapid Detection, to achieve the sample in, the result out.

### BRIEF DESCRIPTION OF THE DRAWINGS

In order to illustrate the technical solutions of the embodiments of the present application more clearly, the following briefly introduces the drawings that are used in the embodiments of the present application. Apparently, the drawings described below are only some embodiments of the present application, and those skilled in the art can also obtain other drawings based on these drawings without creative efforts.

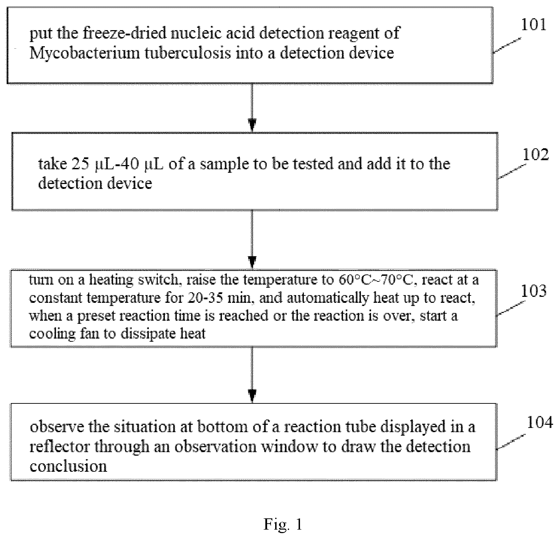

Fig. 1 is a nucleic acid detection method for Mycobacterium tuberculosis provided in an embodiment of the present application.

### DETAILED DESCRIPTION

The following will clearly and completely describe the technical solutions in the embodiments of the present application with reference to the drawings in the embodiments of the present application. Apparently, the described embodiments are only some of the embodiments of the present application, not all of them. Based on the embodiments in the present application, all other embodiments obtained by those skilled in the art without making creative efforts belong to the protection scope of the present application.

In the description of the present application, it should be understood that the orientation or positional relationship indicated by the terms "center", "longitudinal", "transverse", "length", "width", "thickness", "upper", "lower", "front", "rear", "left", "right", "vertical", "horizontal", "top", "bottom", "inner", "outer", etc. is based on the orientation or positional relationship shown in the drawings, and is only for the convenience of describing the present application and simplifying the description. It is not intended to indicate or imply that the device or element referred to must have a particular orientation, be constructed, or operate in a particular orientation, and thus should not be construed as limiting the application. In addition, the terms "first" and "second" are used for descriptive purposes only, and cannot be interpreted as indicating or implying relative importance or implicitly specifying the quantity of indicated technical features. Thus, features defined as "first" and "second" may explicitly or implicitly include one or more features. In the description of the present application, "plurality" means two or more, unless otherwise specifically defined.

In the present application, the word "exemplary" is used to mean "serving as an example, illustration or description". Any embodiment described in the present application as "exemplary" is not necessarily to be construed as preferred or advantageous over other embodiments. The following description is given to enable any person skilled in the art to make and use the present application. In the following description, details are set forth for purposes of explanation. It should be understood that a person skilled in the art would recognize that the present application may be practiced without these specific details. In other instances, well-known structures and processes are not described in detail to avoid obscuring the description of the present application with unnecessary detail. Thus, the present application is not intended to be limited to the embodiments shown, but is to be accorded the widest scope consistent with the principles and features disclosed in the present application.

### Embodiment 1

The present embodiment provides a nucleic acid detection reagent for Mycobacterium tuberculosis, comprising: Tris-HCL, MgCl2, UNG enzyme, Bst DNA polymerase, reverse transcriptase, trehalose, sucrose, mannitol, Tween 20, guanidine hydrochloride and the primers of six kinds of Mycobacterium tuberculosis, the primers of six kinds of Mycobacterium tuberculosis are TB-F3, TB-R3, TB-FIP, TB-BIP, TB-LF and TB-LB, wherein TB is tuberculosis branch bacilli.

Nucleic acid detection reagents can lyse pathogens, release their genomic nucleic acids, and provide an inhibition-resistant buffer system to achieve the effect of exempting nucleic acid extraction. At the same time, the reagents can be stored at room temperature after freeze-drying, thereby reducing the disadvantages of cold chain transportation and increasing application scenarios. Using tween20 and guanidine hydrochloride to lyse the pathogenic nucleic acid; trehalose, sucrose, and mannitol protect the reaction system during the freeze-drying process, which can realize the freeze-dried powder form of the reagent and the storage of the reagent at room temperature;

Further, the nucleic acid detection reagent also comprises: dATP, dGTP, dCTP, dTTP, dUTP.

In the embodiment of the present application, the nucleic acid detection reagent can be used to perform a one-step detection method for the nucleic acid (DNA or RNA) of pathogens present in throat swabs, nasal swabs, saliva, etc., thereby realizing a convenient, efficient and rapid detection, to achieve the sample in, the result out. The tedious step of nucleic acid extraction is eliminated, and the integration of nucleic acid amplification (RT-LAMP) and its detection and result reading is realized. The visualized nucleic acid isothermal amplification device provided by the present application can directly display the result of the LAMP reaction, and then form an integrated device for fully closed LAMP reaction and automatic interpretation of the LAMP result, thereby realizing a convenient, efficient and rapid detection, to achieve the sample in, the result out. Therefore, it has rich application scenarios, which can be used in customs, border inspection, clinics, hospital outpatient and emergency departments, personal home self-test, etc..

Wherein, the sequence information of TB-F3 is ATACGGCCCAGAC; the sequence information of TB-R3 is GTAATTCCGGACAACG; the sequence information of TB-FIP is TACAACCCGAAGGCCGTCATCAGTGGGGAATAT; the sequence information of TB-BIP is TCCGGGTTCTCTCGGATTGAC ACCCTACGTATT; the sequence information of TB-LF is ATCAGGCTTGCGCC, and the sequence information of TB-LB is AACTACGTGCCAGCAG.

In the present embodiment, the primers are designed according to genome nucleic acid sequence of Mycobacterium tuberculosis, so as to obtain the sequence information of the primers.

Wherein, a concentration of TB-F3 is 100 µmol/L, a concentration of TB-R3 is 100 µmol/L, and a concentration of TB-FIP is 100 µmol/L, a concentration of TB-BIP is 100 µmol/L, a concentration of TB-LF is 100 µmol/L and a concentration of TB-LB is 100 µmol/L.

Wherein, a volume of TB-F3 is 0.030 µL-0.050 µL, a volume of TB-R3 is 0.030 µL-0.050 µL, and a volume of TB-FIP is 0.800 µL - 1.300 µL, a volume of TB-BIP is 0.800 µL-1.300 µL, a volume of TB-LF is 0.050 µL-0.080 µL and a volume of TB-LB is 0.050 µL-0.080 µL.

Wherein, a concentration of Tris-HCL is 200 mmol/L, a concentration of MgCl2 is 25mmol/L, a concentration of UNG enzyme is 1 U/µL, a concentration of Bst DNA polymerase is 8000 U/mL, a concentration of reverse transcriptase is 15000 units/mL, a concentration of trehalose is 3 mmol/L, a concentration of sucrose is 5 mmol/L, a concentration of mannitol is 2 mmol/L, a concentration of guanidine hydrochloride is 3 mmol/L.

Wherein, a volume of Tris-HCL is 1.5-2 µL, a volume of MgCl2 is 1-1.5 µL, a volume of UNG enzyme is 0.02-0.05 µL, a volume of Bst DNA polymerizes is 0.3-0.5 µL, a volume of reverse transcriptase is 0.3-0.5 µL, a volume of trehalose is 0.1-0.2 µL, a volume of sucrose is 0.05-0.1 µL, a volume of mannitol is 2-6 µL, a volume of guanidine hydrochloride is 0.05-0.08 µL.

In the present embodiment, the nucleic acid detection reagent is freeze-dried and stored at room temperature.

See Table 1 for the nucleic acid detection reagent raw materials (reaction system raw materials) used in the detection of Mycobacterium tuberculosis.

**Table 1 TB virus nucleic acid detection reagent formula (RT-LAMP method)**

| Raw material and its concentration | Usage amount (volume, µL) |
|---|---|
| Tns-HCL(200mM) | 1.500-2.000 |
| MgCl2 (25 mM) | 1.000-1.500 |
| dATP (100 mM) | 0.020-0.050 |
| dGTP (100 mM) | 0.020-0.050 |
| dCTP (100 mM) | 0.020-0.050 |
| dTTP (100 mM) | 0.020-0.050 |
| dUTP (100 mM) | 0.020-0.050 |
| UNG (1 U/µL) | 0.020-0.050 |
| Bst3.0 DNAPolymerase(8,000 U/mL) | 0.300-0.500 |
| Reverse transcriptase (15,000 units/mL) | 0.300-0.500 |
| TB -F3 (100 µM) | 0.030-0.050 |
| TB -R3 (100 µM) | 0.030-0.050 |
| TB -FIP (100 µM) | 0.800-1.300 |
| TB -BIP (100 µM) | 0.800-1.300 |
| TB -LF (100 µM) | 0.050-0.080 |
| TB -LB (100 µM) | 0.050-0.080 |
| Trehalose (3M) | 0.100-0.200 |
| Sucrose (5M) | 0.050-0.100 |
| Mannitol (2M) | 2.000-6.000 |
| Phenol red (900µM) | 1.000-1.500 |
| Tween 20 (v/v 10%) | 0.0500-0.0800 |
| Guanidine Hydrochloride (3M) | 0.050-0.0800 |

Note: In Table 1, M in mM, µM, and M is the abbreviation of mol/L.

**Table 2 is a list of primer sequences used to detect TB**

| Virus | Primer | Sequence information (primer for gap pol region) | SEQ ID NO |
|---|---|---|---|
| TB | F3 | ATACGGCCCAGAC | 1 |
| | B3 | GTAATTCCGGACAACG | 2 |
| | FIP | TACAACCCGAAGGCCGTCATCAGTGGGGAATAT | 3 |
| | BIP | TCCGGGTTCTCTCGGATTGAC ACCCTACGTATT | 4 |
| | LF | ATCAGGCTTGCGCC | 5 |
| | LB | AACTACGTGCCAGCAG | 6 |

In this embodiment, after the sample to be tested is placed in the sample preservation solution for preservation, the raw material of the reaction reagent in Table 1 is freeze-dried and then placed in the detection device, and the enterprise reference product of Mycobacterium tuberculosis is tested as follows aspects of testing. The enterprise reference product is composed of artificially synthesized Mycobacterium tuberculosis pseudovirus and positive samples of Mycobacterium tuberculosis. The pseudovirus is provided by a certain company and diluted to a working concentration before the experimental test.

The nucleic acid detection reagent is tested, and the specific detection is as follows:

### 01. Detection limit experiment:

Detect the 2 detection limit references in Table 3:

**Table 3 is the detection limit reference product information**

| Reference No. | Concentration | Details |
|---|---|---|
| TB L1 | 200 copy/mL | Pseudovirus provided by a company |
| TB L2 | 1000 copy/mL | Pseudovirus provided by a company |

The detection limit experiment was repeated 20 times for each detection limit reference product.

For the two kinds of detection limit reference products, in 20 repeated tests, all 100% were positive, which proved the sensitivity of the detection system of the present application.

### 02. Conformity rate experiment of negative and positive reference products:

TB P1 positive reference products in Table 4 were tested 3 times, and the test results were all positive for Mycobacterium tuberculosis. The TB N1 negative reference product was tested 3 times, and the test results were all negative for Mycobacterium tuberculosis.

**Table 4 is the negative and positive reference product information**

| Reference No. | Concentration | Details |
|---|---|---|
| TB P1 | 2000 copy/mL | Pseudovirus provided by a company |
| TB N1 | / | Throat swab fluid from normal people (negative for Mycobacterium tuberculosis) |

### 03. Specificity experiment

The specific reference substances in Table 5 were tested 3 times each, and all were positive.

**Table 5 is the information of specific experimental reference products**

| Reference No. | Concentration | Details |
|---|---|---|
| TB T1 | 1000 copy/mL | Pseudovirus provided by a company |
| TB T2 | 2000 copy/mL | Pseudovirus provided by a company |
| TB T3 | 10000 copy/mL | Pseudovirus provided by a company |
| TB T4 | 100000 copy/mL | Pseudovirus provided by a company |

### 04. Precision experiment

Test precision references in Table 6:

**Table 6 is the reference product information for the precision experiment**

| Reference No. | Concentration | Genotype |
|---|---|---|
| TB T1 | 2000 copy/mL | Pseudovirus provided by a company |
| TB T2 | 5000 copy/mL | Pseudovirus provided by a company |
| TB T3 | / | Throat swab fluid from normal people (negative for Mycobacterium tuberculosis virus) |

Test precision reference products TB T1, TB T2, and each precision reference product was tested 10 times, all of which were positive. The test precision reference product TB T3 was tested 10 times, all of which were negative.

### 05. Extraction-free test

Assays for extraction-free test references in Table 7:

**Table 7 the reference product information for extraction-free testing experiments**

| Reference No. | Concentration | Genotype |
|---|---|---|
| TB M1 | 2000 copy/mL | Synthetic pseudovirus |
| TB M2 | 5000 copy/mL | Mycobacterium tuberculosis positive throat swab sample |
| TB M3 | / | Throat swab fluid from normal people (negative for Mycobacterium tuberculosis) |

The extraction-free reference products TB M1 and TB M2 were detected, and each precision reference product was tested 3 times, all of which were positive. The extraction-free reference product TB M3 was tested 3 times, all of which were negative.

### 06. Anti-interference ability experiment

After testing, hemoglobin with a concentration of 2g/L and mucin with a concentration of 20mg/mL in the sample had no effect on the detection. The concentration of drugs listed in Table 8 in the sample has no effect on the test results.

**Table 8 shows the drugs and concentrations that have no effect on the test results in the anti-interference ability experiment**

| Alpha-interferon | Zanami vir | Leigh Bavirin | Oseltamiv ir | Peramivir | Hydrochlo ric acid Histamine | Ritonavir | Abidor |
|---|---|---|---|---|---|---|---|
| 1000 Million U/mL | 50mg/ mL | 2g/mL | 200mg/ mL | 1g/mL | 5mg/mL | 250mg/ mL | 1g/mL |
| Levofloxacin | Azithro mycin | Ceftria xone | Mometaso ne | Fluticasone | Phenylephr ine | Oxymetazoli ne | Beclom ethason e |
| 500mg/mL | 1g/mL | 2g/mL | 1mg/mL | 10mg/mL | 50mg/mL | 0.5mg/mL | 2mg/mL |

### 07. Clinical sample testing

80 throat swab samples that were positive for Mycobacterium tuberculosis virus were all positive, and 210 throat swab samples of normal people were tested negative; All 57 positive Mycobacterium tuberculosis samples were tested positive, and all 256 normal saliva samples were tested negative.

This detection reagent can be applied to the detection of Mycobacterium tuberculosis nucleic acid in throat swabs, nasal swabs, and saliva samples. Through the comparison of the above detection results, it is concluded that the detection reagent and method provided by the present application are applied to the detection of human Mycobacterium tuberculosis and the detection results obtained meet the detection requirements of enterprise standard products, and meet the basic performance requirements of the methodology for the detection of Mycobacterium tuberculosis.

In this embodiment, the raw materials of the nucleic acid detection reagents further comprise fluorescent reagents or fluorescent probes. The above-mentioned lyophilized powder reagent may further comprise a visual chromogen, such as a reaction indicator, a fluorescent reagent or a fluorescent probe. In a specific embodiment of the present application, HBYT is selected as the chromogenic agent for the visual reaction. Before the reaction, it is purple. After the reaction, the positive result is green, and the negative result is still purple. During the reaction process of LAMP, DNA polymerase performs polymerization, changes the number of protons and then changes the pH value, thus turning the reaction liquid into green. The whole reaction process is fast, and the color change is clear and visible to the naked eye. This product can be used in any LAMP or RT-LAMP reaction, only need to provide the sample and heating device, and the result of positive amplification can be read with naked eyes in a short time.

In the present application, after mixing the above-mentioned raw materials in a certain proportion, the dry powder is formed after the freeze-drying program of the freeze-drying machine (model of the freeze-drying machine: LGJ-20F gland type, and the manufacturer is Beijing Songyuan) is run. The nucleic acid isothermal amplification detection reagent provided by the present application can be used for the detection of pathogenic microorganisms for non-disease diagnosis purposes, and can further be made into corresponding detection kits and the like. When the kit is made, it may further comprise a sample preservation solution, which may be physiological saline (0.9% NaCl solution).

Based on the nucleic acid detection reagent of Mycobacterium tuberculosis provided in the foregoing embodiments, the present embodiment provides a nucleic acid detection method of Mycobacterium tuberculosis, and the detection of Mycobacterium tuberculosis based on the aforementioned nucleic acid detection reagent comprises:
Step 101: Put the freeze-dried nucleic acid detection reagent of Mycobacterium tuberculosis into a detection device;
Step 102: Take 25 µL-40 µL of a sample to be tested and add it to the detection device;
Step 103: Turn on a heating switch, raise the temperature to 60°C~70°C, react at a constant temperature for 20-35 min, and automatically heat up to react, when a preset reaction time is reached or the reaction is over, start a cooling fan to dissipate heat;
Step 104: Observe the situation at the bottom of the reaction tube displayed in the reflector through the observation window, and drawing a detection conclusion.

Specifically, the sample to be tested in the present application can be collected and stored in the following manner.

Throat swab sample collection: insert the swab (material of swab handle: ABS plastic, material of swab head: nylon flocking material) from the oral cavity into the throat (or nasal cavity), across the base of the tongue to the posterior pharyngeal wall or the back of the uvula, wipe the patient's posterior pharyngeal wall and tonsils on both sides with appropriate force, and take out the swab to avoid touching the tongue, oral mucosa and saliva. Indicate the time when the specimen was collected and submit it for inspection in time. Insert the collected swab into the sample preservation solution, and rotate it against the inner wall of the test tube for about 10 times, so that the sample can be dissolved in the preservation solution of the dropping bottle as much as possible. Squeeze the head of the swab along the inner wall of the sample preservation liquid tube to keep the liquid in the tube as much as possible, take out the swab, and tighten the cap of the tube. The processed throat swab fluid is the sample to be tested.

Nasal swab sample collection: insert the swab (material of swab handle: ABS plastic, material of swab head: nylon flocking material) from the nasal cavity into the nasal cavity completely, reach the nasopharynx, wipe with appropriate force, take out the swab and put it into the nasal cavity in the dropper bottle. Insert the collected swab into the sample preservation solution, and rotate it against the inner wall of the test tube for about 10 times, so that the sample can be dissolved in the preservation solution of the dropping bottle as much as possible. Squeeze the head of the swab along the inner wall of the sample preservation liquid tube to keep the liquid in the tube as much as possible, take out the swab, and tighten the cap of the tube. The processed nasal swab fluid is the sample to be tested.

Saliva sample collection: Open the cap of the dropper bottle, spit about 1 mL of saliva into the dropper bottle, close the cap, and gently mix the saliva sample. The processed throat swab fluid is the sample to be tested.

The use process of the novel visualized nucleic acid isothermal amplification device provided by the present application can be as follows: Open the top cover, take 25 µL-40 µL of the sample to be tested and add it to the reaction tube. The reaction tubes are prefilled with lyophilized powders of all detection reagents. Close the lid of the reaction tube tightly (the reaction tube can be a centrifuge tube commonly used for nucleic acid amplification on the market), and place it in the fixing hole of the reaction tube. Start the heating switch, adjust the target heating temperature and time (heating to 60°C∼70°C, the best is 65°C, constant temperature reaction for 20-35min, the best is 20min), automatically heat the reaction,, and when the preset reaction time is reached or the reaction is over, start the cooling fan to dissipate heat. Observe the situation at the bottom of the reaction tube (that is, the final color of the reaction solution) displayed in the reflector through the observation window, and draw a conclusion (nucleic acid negative or positive).

The detection system in the present application has rich application scenarios, and can be used for home nucleic acid self-test, clinic nucleic acid detection, customs, border inspection, hospital outpatient and emergency detection, pediatric emergency, pet hospital, etc.

The above description is only the implementation manner of the present application, and does not limit the patent scope of the present application. Any equivalent structure or equivalent process transformation made by using the contents of the description and drawings of this application, or directly or indirectly used in other related technical fields, is also included in the protection scope of the present application.

## Claims

1. A nucleic acid detection reagent for Mycobacterium tuberculosis, comprising: Tris-HCL, MgCl2, UNG enzyme, Bst DNA polymerase, reverse transcriptase, trehalose, sucrose, mannitol, Tween 20, guanidine hydrochloride and the primers of six kinds of Mycobacterium tuberculosis, the primers of six kinds of Mycobacterium tuberculosis are TB-F3, TB-R3, TB-FIP, TB-BIP, TB-LF and TB-LB, wherein TB is tuberculosis branch bacilli.

2. The nucleic acid detection reagent for Mycobacterium tuberculosis according to claim 1, wherein the sequence information of TB-F3 is ATACGGCCCAGAC; the sequence information of TB-R3 is GTAATTCCGGACAACG; the sequence information of TB-FIP is TACAACCCGAAGGCCGTCATCAGTGGGGAATAT; the sequence information of TB-BIP is TCCGGGTTCTCTCGGATTGAC ACCCTACGTATT; the sequence information of TB-LF is ATCAGGCTTGCGCC, and the sequence information of TB-LB is AACTACGTGCCAGCAG.

3. The nucleic acid detection reagent for Mycobacterium tuberculosis according to claim 1, wherein the primers are designed according to genome nucleic acid sequence of Mycobacterium tuberculosis, so as to obtain the sequence information of the primers.

4. The nucleic acid detection reagent for Mycobacterium tuberculosis according to claim 1, wherein a concentration of TB-F3 is 100 µmol/L, a concentration of TB-R3 is 100 µmol/L, and a concentration of TB-FIP is 100 µmol/L, a concentration of TB-BIP is 100 µmol/L, a concentration of TB-LF is 100 µmol/L and a concentration of TB-LB is 100 µmol/L.

5. The nucleic acid detection reagent for Mycobacterium tuberculosis according to claim 4, wherein a volume of TB-F3 is 0.030 µL-0.050 µL, a volume of TB-R3 is 0.030 µL-0.050 µL, and a volume of TB-FIP is 0.800 µL - 1.300 µL, a volume of TB-BIP is 0.800 µL-1.300 µL, a volume of TB-LF is 0.050 µL-0.080 µL and a volume of TB-LB is 0.050 µL-0.080 µL.

6. The nucleic acid detection reagent for Mycobacterium tuberculosis according to claim 5, wherein a concentration of Tris-HCL is 200 mmol/L, a concentration of MgCl2 is 25mmol/L, a concentration of UNG enzyme is 1 U/µL, a concentration of Bst DNA polymerase is 8000 U/mL, a concentration of reverse transcriptase is 15000 units/mL, a concentration of trehalose is 3 mmol/L, a concentration of sucrose is 5 mmol/L, a concentration of mannitol is 2 mmol/L, a concentration of guanidine hydrochloride is 3 mmol/L.

7. The nucleic acid detection reagent for Mycobacterium tuberculosis according to claim 6, wherein a volume of Tris-HCL is 1.5-2 µL, a volume of MgCl2 is 1-1.5 µL, a volume of UNG enzyme is 0.02-0.05 µL, a volume of Bst DNA polymerizes is 0.3-0.5 µL, a volume of reverse transcriptase is 0.3-0.5 µL, a volume of trehalose is 0.1-0.2 µL, a volume of sucrose is 0.05-0.1 µL, a volume of mannitol is 2-6 µL, a volume of guanidine hydrochloride is 0.05-0.08 µL.

8. The nucleic acid detection reagent for Mycobacterium tuberculosis according to claim 1, wherein the nucleic acid detection reagent is freeze-dried and stored at room temperature.

9. The nucleic acid detection reagent for Mycobacterium tuberculosis according to claim 1, wherein the raw material of the nucleic acid detection reagent further includes a fluorescent reagent or a fluorescent probe.

10. A nucleic acid detection method for Mycobacterium tuberculosis, wherein a detection of Mycobacterium tuberculosis based on the nucleic acid detection reagent according to claim 1 comprising:
put the freeze-dried nucleic acid detection reagent of Mycobacterium tuberculosis into a detection device;
take 25 µL-40 µL of a sample to be tested and add it to the detection device;
turn on a heating switch, raise the temperature to 60°C∼70°C, react at a constant temperature for 20-35 min, and automatically heat up to react, when a preset reaction time is reached or the reaction is over, start a cooling fan to dissipate heat;
observe the situation at bottom of a reaction tube displayed in a reflector through an observation window to draw the detection conclusion.
